# EUROPEAN PATENT SPECIFICATION

(11) **EP 4 525 950 B1**
(45) Date of publication and mention of the grant of the patent: **04.11.2026**
(21) Application number: 23730204.7
(22) Date of filing: 12.05.2023
(51) Int. Cl.: A61M 5/20, A61M 5/24, A61M 5/32, A61M 5/34

(54) **INJECTION DEVICE WITH A NEEDLE DETECTION FEATURE**
INJEKTIONSGERÄT MIT NADELDETEKTIONSFUNKTION
DISPOSITIF D'INJECTION AVEC DÉTECTION DE L'AIGUILLE

(30) Priority: 16.05.2022 US 202263364748 P
(43) Date of publication of application: 26.03.2025
(60) Divisional application: 25182195.5 / 4 606 405; 25182300.1 / 4 606 406
(73) Proprietor: Pfizer Inc., New York, NY 10001-2192 (US)
(72) Inventor: COHEN, Ziv, Gesher Haziv, 228150 West Galilee (IL); DOUMANI, Richard Walter, Notting Hill, Victoria 3168 (AU); KACHLER, Ilan, 2221608 Nahariya (IL); NAAMAN, Idan, Kibbutz Farod, 2011000 Upper Galilee (IL); PORAT, Amotz, Kibbutz Cabri, 2512000 West Galilee (IL)
(74) Representative: Plasseraud IP
(86) International application number: PCT/IB2023/054936
(87) International publication number: WO 2023/223165

(56) References cited:
- EP-A1- 2 674 184
- US-A1- 2003 040 725
- US-A1- 2007 197 968
- US-A1- 2007 293 825
- US-A1- 2016 296 700

## Description

### Technical field

The present invention relates to injection devices, in particular to electromechanical injection devices for the injection of a medicament from a replaceable cartridge received in the device. Such injection devices are typically controlled by an electronic control system. Also, such injection devices require that a needle is attached to the cartridge before use and detached and disposed of after use.

More specifically, the invention relates to an injection device comprising a needle sensing feature for detecting the attachment of a needle to the cartridge and providing a corresponding information to the electronic control system.

In a second aspect, the invention relates to an injection device comprising a needle hiding element telescopically mounted in the housing for hiding a needle attached to the cartridge.

In a third aspect, the invention relates to an injection device having a position sensing feature for detecting a condition of the device being pressed against an injection surface.

### Background of the invention

In an electronically controlled injection device of this type, it is desirable that the operation of the device for injecting the medicament to the user be prevented if the condition of a needle correctly attached to the cartridge is not met. It is therefore desirable that the device includes means for detecting the presence of a needle and the correct attachment to the cartridge.

Systems for detecting the presence of a needle are known in the art, for example in WO 2005/077441, wherein the detection system uses optical sensors located in the area of the cartridge/needle interface. Further examples are known from EP 2 674 184, US 2016/296700, and US 2007/197968.

The use of such optical sensing means has several drawbacks, in particular the location of the optical transmitters and receivers, that typically implies that the sensing means cannot be grouped with the other electronic components. Also, optical sensing means may be subject to various disturbances that may affect the reliability of the detection.

It is therefore an objective of the invention to provide a sensing feature based on an alternative technology, that would be less complex to implement in an injection device and that would have an increased robustness.

It is a further objective of the invention to provide a needle hiding feature that can be conveniently handled for the ease of the operation of attaching a needle to the cartridge.

It is still a further objective of the invention to provide a position sensing feature involving simple and reliable means that are easy to implement in the device.

### Summary of the Invention

According to the present invention, it is provided an injection device comprising
- an elongated housing extending along a longitudinal axis and configured to receive a medicament cartridge;
- a needle connection for connection to a needle assembly, including a needle and a needle hub, and thus attaching the needle to the cartridge;
- an injection drive mechanism for driving a piston within the cartridge and thus expel medicament from the cartridge;
- an electronic control system for controlling the injection drive mechanism based on user input and information indicative of the status of the device; and
- a needle sensing feature for detecting the attachment of a needle to the cartridge and providing a corresponding information to the electronic control system,
wherein the needle sensing feature includes
- a slider which is axially movable relative to the housing between an extended position and a retracted position, the slider comprising a magnetic part and being biased into its extended position and maintained in its retracted position when a needle is attached to the cartridge;
- an electromagnetic sensor fixedly arranged within the housing, such that the magnetic part can move with the slider between positions remote from the electromagnetic sensor and a position in the vicinity of the electromagnetic sensor when the slider is in its retracted position, wherein the electromagnetic sensor is caused to generate, and provide to the electronic control system, a signal indicative of a needle being attached to the cartridge.

Preferred embodiments may include one or several of the following features:
- the electronic control system is adapted to prevent actuation of the injection drive mechanism for expelling medicament from the cartridge, unless it is provided with a signal indicative of a needle being attached to the cartridge by the electromagnetic sensor;
- the injection drive mechanism comprises an axially displaceable piston rod for driving a piston in the cartridge and an electrical motor for displacing the piston rod, the electrical motor being controlled by the electronic control system;
- the injection device further comprises a chassis arranged within the housing and fixedly attached thereto, and the injection drive mechanism is attached to the chassis;
- the electronic control system includes a processor and a printed circuit board fixedly arranged within the housing, wherein the electromagnetic sensor is supported by the printed circuit board and connected to the processor so as to provide to the processor a signal indicative of a needle being attached to the cartridge;
- the injection device further comprises a cartridge holder for receiving and holding the cartridge, the cartridge holder being movable relative to the housing between an open position, wherein a cartridge can be placed within or removed from the cartridge holder, and a closed position, wherein the cartridge is in a position suitable for injection;
- the cartridge holder is pivotable between the open position and the closed position;
- the chassis and the cartridge holder comprise fitting pivot pins and pivot bearings forming a hinge connection, whereby the cartridge holder is pivotable with respect to the housing between the open and the closed position;
- the needle connection comprises a threaded tubular section of the cartridge holder provided at a distal end thereof for threaded connection to the needle hub;
- the slider comprises a base for engagement with the needle assembly, the base having a central bore designed such that the needle can pass through the bore while the needle hub engages the base at a peripheral rim of the bore when a needle assembly is being connected to the needle connection;
- the base of the slider has a cup shape with a bottom wall provided with the bore and proximally oriented, the bottom wall being substantially flat whereby, when the needle hub is threadedly connected to the threaded section of the cartridge holder and the needle thus attached to the cartridge, the bottom wall is tightly maintained in the retracted position of the slider between the needle hub and the cartridge holder, thereby providing partial sealing preventing ingress of any expelled medicament from the cartridge into the housing;
- the slider comprises an axial arm proximally projecting from the base and provided with the magnetic part;
- the slider comprises guiding elements proximally projecting from the base for axially guiding the slider as it moves between the extended position and the retracted position;
- the chassis comprises complementary guiding elements engaging with respective guiding elements of the slider for axially guiding the slider as it moves between the extended position and the retracted position;
- the slider comprises biasing springs operatively associated with the respective guiding elements for biasing the slider into its extended position.

According to a further aspect of the present disclosure it is provided an injection device comprising
- an elongated housing extending along a longitudinal axis and configured to receive a medicament cartridge;
- an injection drive mechanism for driving a piston within the cartridge and thus expel medicament from the cartridge;
- a needle hiding element, which is telescopically mounted in the housing and can be axially displaced between a fully extended position, wherein the needle hiding element distally protrudes from the housing and that is suitable for hiding a needle attached to the cartridge, and a fully retracted position proximally spaced from the extended position corresponding to the condition of pressing the device against an injection site for injecting the medicament; and
- a latch mechanism that can be actuated by a user for releasably locking the needle hiding element in one partially retracted position, proximally spaced from the fully extended position and distally spaced from the fully retracted position, said partially retracted position being suitable for facilitating attaching a needle to the cartridge,

wherein the latch mechanism includes a latch button that is actuatable by the user to be displaced between a release position and a locking position, a first latch member attached to the latch button and a corresponding second latch member provided on the needle hiding element, the displacement of the needle hiding element defining a sliding path for the second latch member,
and wherein the latch mechanism is designed such that the first latch member is out of the sliding path of the second latch member when the latch button is in the release position and in said sliding path when the latch button is in the locking position, and such that, in the locking position, the first and second latch members engage to prevent distal displacement of the needle hiding element from the partially retracted position but allow for proximal displacement of the needle hiding element.

Preferred embodiments may include one or several of the following features:
- the latch button outwardly protrudes from the housing and is transversally displaceable between the release and locking positions;
- one of the first and second latch members includes a rigid stop member formed with a transversal abutment surface and an inclined ramp and the other of the first and second latch members includes a flexible arm suitable, in the locking position, to engage with the ramp and flex out to allow for proximal displacement of the needle hiding element and to engage with the abutment surface to prevent distal displacement of the needle hiding element from the partially retracted position;
- the flexible arm is attached to the latch button and the rigid stop member is provided on the needle hiding element;
- the flexible arm is a metallic blade extending in an axial direction and attached at one end to the latch button, the other end of the blade being a free end having an edge engaging with the transversal abutment surface in the locking position;
- the injection device further comprises biasing means for biasing the needle hiding element into its fully extended position;
- the needle hiding element comprises an essentially cylindrical wall suitable, in the fully extended position, for hiding a needle attached to the medicament cartridge, and proximally projecting from the cylindrical wall, an axial arm provided with the second latch member;
- the needle hiding element further comprises guiding elements proximally projecting from the cylindrical wall for axially guiding the needle hiding element as it moves between the fully extended position and the fully retracted position;
- the needle hiding element further comprises at the distal end of the cylindrical wall an enlarged gripping portion that radially extends beyond the cylindrical wall, said enlarged portion engaging a distal edge of the housing in the fully retracted position and thus preventing further retraction of the needle hiding element;
- the gripping portion has a ridged surface for enhancing prehension by a user;
- the needle hiding element further comprises an axially extending retention arm proximally projecting from the cylindrical wall and provided with a stop member suitable for engaging with a complementary stop member secured to the housing, in the fully extended position, to limit the displacement of the needle hiding element in the distal direction;
- the injection device further comprises
   - an electronic control system for controlling the injection drive mechanism based on user input and information indicative of the status of the device; and
   - a position sensing feature for detecting the position of the needle hiding element and providing a corresponding information to the electronic control system,
   wherein the position sensing feature includes a magnetic part provided on the needle hiding element and at least a first electromagnetic sensor fixedly arranged within the housing, such that the magnetic part can move with the needle hiding element between positions remote from the first electromagnetic sensor and a position in the vicinity of the first electromagnetic sensor when the needle hiding element is in its fully retracted position, wherein the first electromagnetic sensor is caused to generate, and provide to the electronic control system, a signal indicative of the injection device being pressed against an injection surface and ready for injection;
- the needle hiding element further comprises an axially extending detection arm proximally projecting from the cylindrical wall and provided with the magnetic part;
- the device further comprises a second electromagnetic sensor fixedly arranged within the housing and axially spaced in the distal direction from the first electromagnetic sensor by a distance corresponding to the travel of the needle hiding element between the fully retracted position and said one partially retracted position, wherein the second electromagnetic sensor is caused to generate and provide to the electronic control system, when the magnetic part is in the vicinity of the second electromagnetic sensor, a signal indicative of the needle hiding element being in a suitable position for the user to attach a needle to the cartridge;
- the device further comprises a chassis arranged within the housing and fixedly attached thereto, and wherein the injection drive mechanism is attached to the chassis;
- the chassis comprises complementary guiding elements engaging with respective guiding elements of the needle hiding element for axially guiding the needle hiding element as it moves between the fully extended position and the fully retracted position;
- the electronic control system includes a processor and a printed circuit board fixedly arranged within the housing, wherein the electromagnetic sensor is supported by the printed circuit board and connected to the processor so as to provide to the processor a signal indicative of the position of the needle hiding element;
- the electronic control system is adapted to prevent actuation of the injection drive mechanism for expelling medicament from the cartridge, unless it is provided by the electromagnetic sensor with a signal indicative of the injection device being pressed against an injection surface and ready for injection; and
- the injection drive mechanism comprises an axially displaceable piston rod for driving a piston in the cartridge and an electrical motor for displacing the piston rod, the electrical motor being controlled by the electronic control system.

According to a further aspect of the present disclosure, it is provided an injection device comprising
- an elongated housing extending along a longitudinal axis and configured to receive a medicament cartridge;
- an injection drive mechanism for driving a piston within the cartridge and thus expel medicament from the cartridge;
- a needle hiding element, which is telescopically mounted in the housing and can be axially displaced between a fully extended position, wherein the needle hiding element distally protrudes from the housing and that is suitable for hiding a needle attached to the cartridge, and a fully retracted position proximally spaced from the extended position corresponding to the condition of pressing the device against an injection site for injecting the medicament; and
- an electronic control system for controlling the injection drive mechanism based on user input and information indicative of the status of the device,

wherein the injection device further includes a position sensing feature for detecting the position of the needle hiding element and providing a corresponding information to the electronic control system,
wherein the position sensing feature includes a magnetic part provided on the needle hiding element and at least a first electromagnetic sensor fixedly arranged within the housing, such that the magnetic part can move with the needle hiding element between positions remote from the first electromagnetic sensor and a position in the vicinity of the first electromagnetic sensor when the needle hiding element is in its fully retracted position, wherein the first electromagnetic sensor is caused to generate, and provide to the electronic control system, a signal indicative of the injection device being pressed against an injection surface and ready for injection.

Preferred embodiments may include one or several of the following features:
- the electronic control system is adapted to prevent actuation of the injection drive mechanism for expelling medicament from the cartridge, unless it is provided by the electromagnetic sensor with a signal indicative of the injection device being pressed against an injection surface and ready for injection;
- the injection drive mechanism comprises an axially displaceable piston rod for driving a piston in the cartridge and an electrical motor for displacing the piston rod, the electrical motor being controlled by the electronic control system;
- the injection device further comprises a chassis arranged within the housing and fixedly attached thereto, and the injection drive mechanism is attached to the chassis;
- the electronic control system includes a processor and a printed circuit board fixedly arranged within the housing, wherein the electromagnetic sensor is supported by the printed circuit board and connected to the processor so as to provide to the processor a signal indicative of the position of the needle hiding element;
- the injection device further comprises biasing means for biasing the needle hiding element into its fully extended position;
- the needle hiding element comprises an essentially cylindrical wall suitable, in the fully extended position, for hiding a needle attached to the medicament cartridge, and proximally projecting from the cylindrical wall, an axially extending detection arm provided with the magnetic part;
- the needle hiding element further comprises guiding elements proximally projecting from the cylindrical wall for axially guiding the needle hiding element as it moves between the fully extended position and the fully retracted position;
- the chassis comprises complementary guiding elements engaging with respective guiding elements of the needle hiding element for axially guiding the needle hiding element as it moves between the fully extended position and the fully retracted position;
- the needle hiding element further comprises at the distal end of the cylindrical wall an enlarged gripping portion that radially extends beyond the cylindrical wall, said enlarged portion engaging a distal edge of the housing in the fully retracted position and thus preventing further retraction of the needle hiding element;
- the gripping portion has a ridged surface for enhancing prehension by a user;
- the needle hiding element further comprises an axially extending retention arm proximally projecting from the cylindrical wall and provided with a stop member suitable for engaging with a complementary stop member secured to the housing, in the fully extended position, to limit the displacement of the needle hiding element in the distal direction;
- the device further comprises a latch mechanism that can be actuated by a user for releasably locking the needle hiding element in one partially retracted position, proximally spaced from the fully extended position and distally spaced from the fully retracted position, said partially retracted position being suitable for facilitating attaching a needle to the cartridge,

wherein the latch mechanism includes a latch button that is actuatable by the user to be displaced between a release position and a locking position, a first latch member attached to the latch button and a corresponding second latch member provided on the needle hiding element, the displacement of the needle hiding element defining a sliding path for the second latch member,
and wherein the latch mechanism is designed such that the first latch member is out of the sliding path of the second latch member when the latch button is in the release position and in said sliding path when the latch button is in the locking position, and such that, in the locking position, the first and second latch members engage to prevent distal displacement of the needle hiding element from the partially retracted position but allow for proximal displacement of the needle hiding element;
   - the latch button outwardly protrudes from the housing and is transversally displaceable between the release and locking positions;
   - one of the first and second latch members includes a rigid stop member formed with a transversal abutment surface and an inclined ramp and the other of the first and second latch members includes a flexible arm suitable, in the locking position, to engage with the ramp and flex out to allow for proximal displacement of the needle hiding element and to engage with the abutment surface to prevent distal displacement of the needle hiding element from the partially retracted position;
   - the flexible arm is attached to the latch button and the rigid stop member is provided on the needle hiding element;
   - the flexible arm is a metallic blade extending in an axial direction and attached at one end to the latch button, the other end of the blade being a free end having an edge engaging with the transversal abutment surface in the locking position; and
   - the device further comprises a second electromagnetic sensor fixedly arranged within the housing and axially spaced in the distal direction from the first electromagnetic sensor by a distance corresponding to the travel of the needle hiding element between the fully retracted position and said one partially retracted position, wherein the second electromagnetic sensor is caused to generate and provide to the electronic control system, when the magnetic part is in the vicinity of the second electromagnetic sensor, a signal indicative of the needle hiding element being in a suitable position for the user to attach a needle to the cartridge.

### Brief Description of the Drawings

A preferred embodiment of the invention will now be described in more details, with reference to the following drawings wherein:
- Figure 1 is an exploded perspective view of an injection device according to a preferred embodiment of the invention and a medicament cartridge for use with the device;
- Figures 2A and 2B are perspective views of the device of Figure 1, respectively showing the front and the back sides of the device, in an initial configuration (before use);
- Figure 3 is a cross-sectional view, in an axial plane, of the device in the configuration represented on Figures 2A and 2B;
- Figures 4A and 4B are enlarged views, respectively perspective and elevation views, of the needle hiding element shown on Figure 1;
- Figure 5A is an enlarged perspective view of the slider shown on Figure 1;
- Figure 5B is a bottom view of the slider of Figure 5A;
- Figure 6 is a partial broken away elevation view of the device of Figures 2A and 2B, in the initial configuration, illustrating details of the device;
- Figure 7 is a partial broken away elevation view of the device of the preceding Figures, in an injection configuration, illustrating further details of the device;
- Figure 8 is a perspective view illustrating the introduction of a medicament cartridge in the device of Figures 1-7, or the removal of a used medicament cartridge from the device;
- Figure 9A is a similar cross-sectional view to Figure 3, in a configuration where the needle hiding element is partially retracted and locked for attachment of a needle;
- Figure 9B is a partial broken away elevation view of the device in the configuration of Figure 9A, illustrating the position of the latch member;
- Figure 9C is a partial broken away perspective view of the device in the configuration of Figure 9A, illustrating the relative positions of the slider and the corresponding electromagnetic sensor;
- Figure 9D is a side detail view of the device in the configuration of Figure 9A, illustrating the locking position of the latch button;
- Figure 10A is a similar cross-sectional view to Figures 3 and 9A, in a configuration where the needle hiding element is partially retracted and locked and where a needle is attached to the medicament cartridge;
- Figure 10B is a partial broken away elevation view of the device in the configuration of Figure 10A, the cap of the needle being removed, illustrating the relative positions of the needle hiding element and the corresponding electromagnetic sensors;
- Figure 10C is a partial broken away perspective view of the device in the configuration of Figure 10A, the cap of the needle being removed, illustrating the relative positions of the slider and the corresponding electromagnetic sensor;
- Figure 11A is a similar cross-sectional view to Figure 10A, in a 'ready-to-use' configuration where the needle hiding element is extended and released and where a needle is attached to the medicament cartridge;
- Figure 11B is a similar view to Figure 9D, illustrating the released position of the latch button;
- Figure 12A is a similar cross-sectional view to Figure 11A, in a injection configuration wherein the device is pressed on an injection site with a needle attached to the medicament cartridge, the needle hiding element being fully retracted;
- Figure 12B is a perspective view of the device in the configuration of Figure 12A;
- Figure 12C is a partial broken away elevation view of the device in the configuration of Figures 12A and 12B, illustrating the relative positions of the needle hiding element and the corresponding electromagnetic sensors.

### Detailed Description of a Preferred Embodiment

### Definitions

The following definitions will be used in the present description and claims:
- the term "distal" refers to a location or direction that is close to, or oriented toward, the outlet port for the content of the medicament cartridge when received in the injection device; and
- the term "proximal" refers to a location or direction opposing to the distal location or direction, and/or that is close to, or oriented toward, the prehension part of the injection device.

The invention will now be further illustrated by the following preferred embodiment shown on the Figures.

**The** injection device depicted on the Figures is an electromechanical device that is electronically controlled as, in particular, the injection force is provided by an electrical motor controlled by an electronic control system. **The** device is reusable and adapted to receive a replaceable medicament cartridge containing the medicament to be injected.

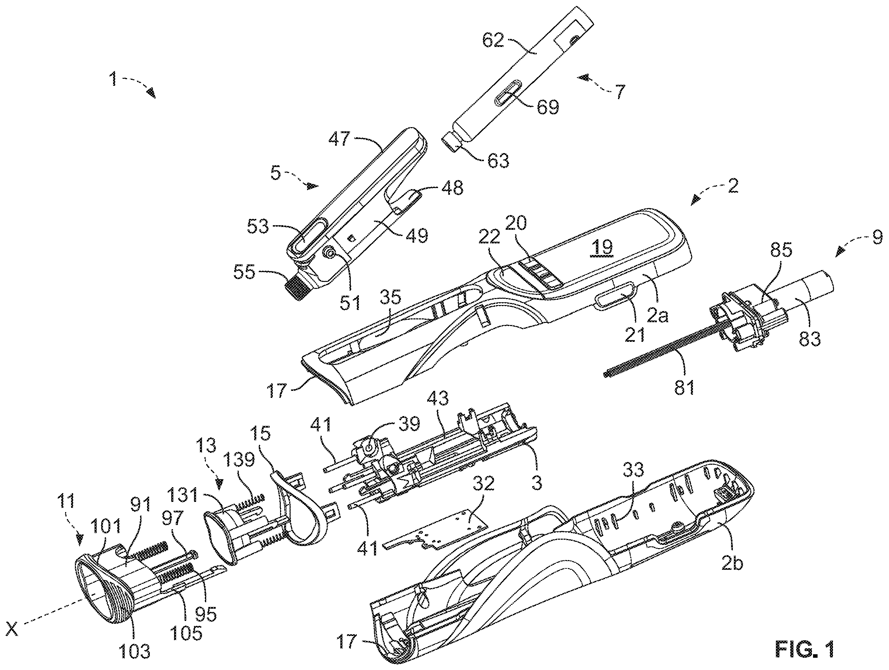

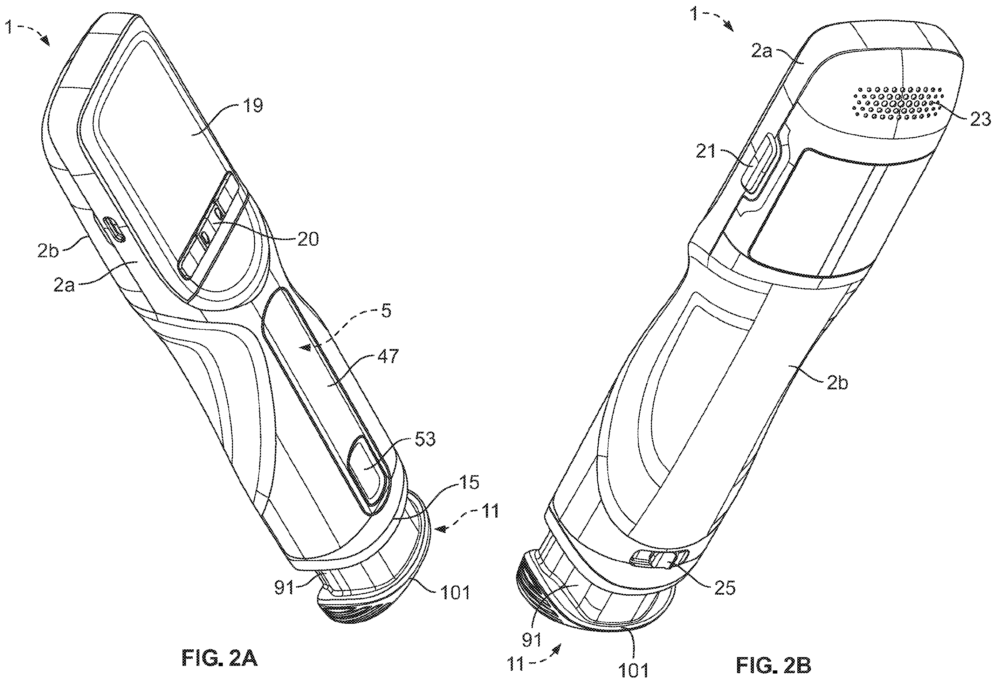

With reference to Figures 1, 2A and 2B, the injection device 1 comprises an elongated housing 2 extending along a longitudinal axis X, the housing 2 being formed by two shells 2a, 2b that fit together to define an internal volume.

**The** injection device 1 further comprises a chassis 3, arranged within the internal volume defined by the shells 2a, 2b and fixedly attached to the housing 2, a cartridge holder 5 (also referred to as a door) for receiving and holding a medicament cartridge 7, and an injection drive mechanism 9 for driving a piston within the cartridge 7 and thus expel medicament from the cartridge.

At a distal end, the device 1 includes a needle hiding element 11 for hiding a needle attached to the cartridge 7, a slider 13 for the detection of the presence of a needle, and an annular seal 15 attached to the housing 2 for achieving a sealed connection between a distal edge 17 of the housing 2 and the needle hiding element when the device 1 is pressed against an injection site.

**The** device 1 also includes a screen 19, provided on a proximal face of the housing 2, for displaying visual information for the user e.g. relating to the status of the device, and menu buttons 20 adjacent to the screen 19, e.g. to select information displayed on the screen 19. **The** device further comprises a switch button 21 for switching on/off the device, provided on a side of the housing 2, an injection button 22 for the user to trigger an injection, and a speaker 23 located at a proximal end for delivering audible signals to the user.

As can be seen on Figure 2B, the device 1 comprises, protruding from the housing 2, a latch button 25 that is actuatable by the user to be transversally displaced for selectively locking or releasing the needle hiding element 11, as will be further described in the following.

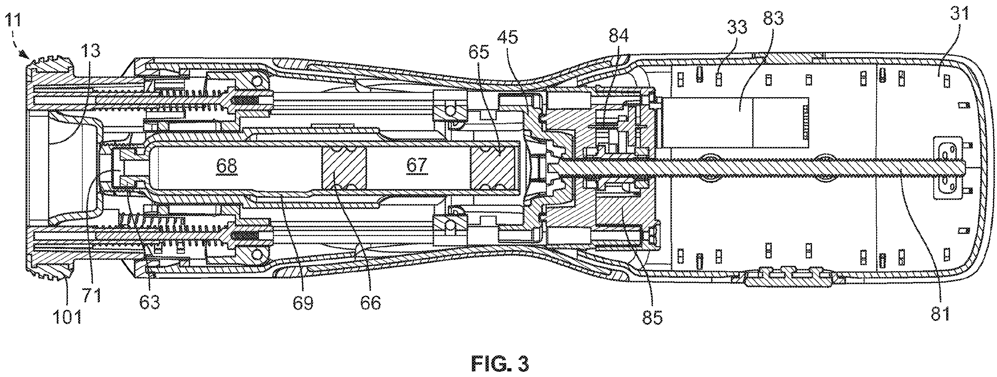

With particular reference to Figures 1 and 3, the device 1 comprises an electronic control system includes a processor (not represented), a main printed circuit board (PCB) 31, fixedly arranged within the housing 2 in a proximal area and supporting the processor, and a second PCB 32 for supporting sensors that will be described in the following. The electronic control system is suitable for acquiring information in particular indicative of the status of the device (e.g. needle attached, device pressed on the injection site) and of user input (e.g. control button 21 pressed) and for controlling the injection drive mechanism based on such information and user input.

It will be seen that the internal surface of the shell 2b is provided with lugs 33 for supporting and fixing the main PCB 31. Also, the shell 2a is formed with an axially elongated through hole 35 designed to fit with the cartridge holder 5.

The chassis 3 is a rigid structural part that is designed to fixedly support a stationary part of the injection drive mechanism 9. The chassis is also designed to pivotally support the cartridge holder 5 and guide the needle hiding element 11 and the slider 13, both mounted onto the chassis 3 so as to axially slide with respect to the housing 2. To that end, the chassis 3 comprises, at the distal end thereof, a pair of opposing pivot bearings 39 having a common transversal axis for pivotally support the cartridge holder 9 and two pairs of axially extending guiding studs 41. One pair of those guiding studs 41 is provided for axially guiding the needle hiding element 11 and the other pair is provided for axially guiding the slider 13, as the needle hiding element 11 and the slider 13, respectively, are moving with respect to the housing 2. The chassis 3 defines over substantially its whole length, a central cavity 43 axially elongated for accommodating the cartridge holder 5 in its closed position and a cartridge 7 inserted therein.

The device 1 comprises means to maintain the cartridge 7 thus received in the housing 2 in a fixed position, in particular in a fixed axial position, with respect to the chassis 3. Those means include a cartridge seal 45 (visible on Figure 3) provided to sealingly engage the distal end of the cartridge to prevent any leakage of medicament into the device.

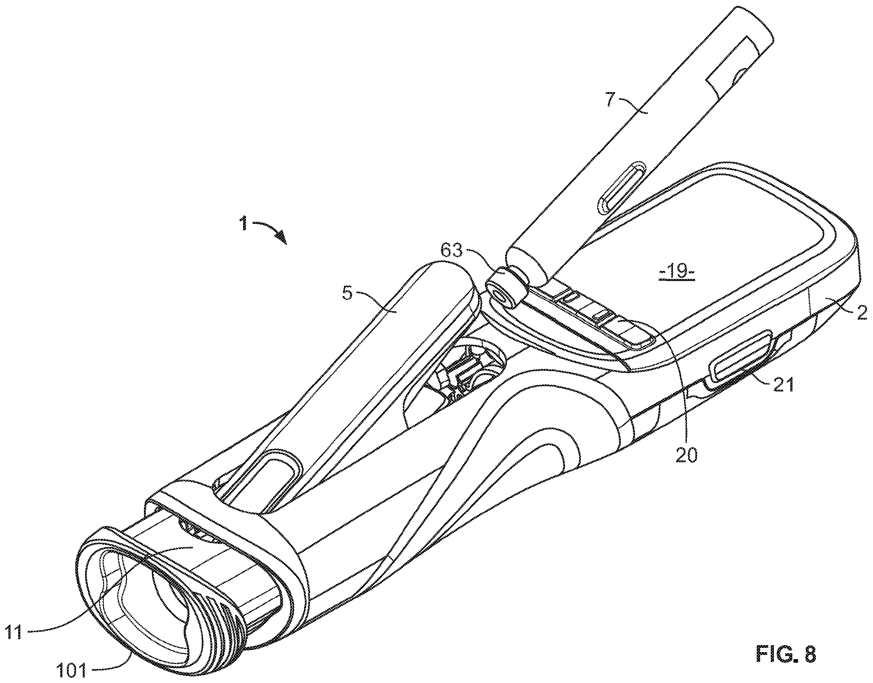

The cartridge holder 5 is formed as an elongated sleeve suitable for axially receiving the medicament cartridge 7 and comprises a top wall 47, a bottom wall 48 and opposing side walls 49. The side walls 49 are provided with respective transversally extending and coaxial pivot pins 51, fitting with the respective pivot bearings 39 of the chassis to form a hinge connection. The cartridge holder 5 is thus rotationally articulated onto the chassis 3, whereby the cartridge holder 5 is pivotable with respect to the housing 2 between an open (Figure 8) and a closed (Figure 2A) position. In the open position, a cartridge 7 can be placed within or removed from the cartridge holder 5. In the closed position, a cartridge 7 received in the cartridge holder 5 is in a position suitable for injection. The cartridge holder 5 is shaped and dimensioned to pass through the elongated through hole 35 of the shell 2a. In particular, the top wall 47 is shaped so as to fit with and entirely close the elongated through hole 35 of the shell 2a when the cartridge holder 5 is in the closed position.

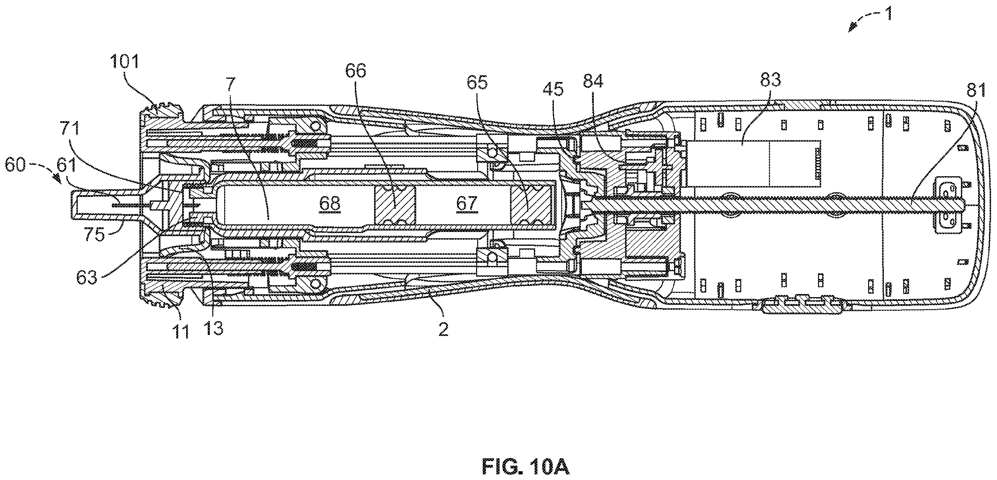

The cartridge holder 5 is provided on its top wall 47 with a window 53 for visual inspection of the content of the cartridge 7. Also, the cartridge holder 5 is formed at its distal end with a needle connection 55 for connection to a needle assembly 60 (Figure 10A) and attachment of a needle 61 (Figure 10A) to the medicament cartridge 7. The needle connection 55 is formed by a threaded section of a distally projecting cylindrical sleeve provided to accommodate the distal end of the medicament cartridge 7.

The cartridge holder 5 is provided with a door magnet (not shown) attached to the bottom wall 48 for the detection of the closed position of the cartridge holder 5 by a door sensor arranged within the housing 2. The bottom wall 48 is also formed with an elongated through hole engageable by a flexible detection arm attached to the chassis 3 for the detection of the presence of a cartridge by a cartridge sensor arranged within the housing 2.

The mechanism for latching the cartridge holder 5 in the closed position and for opening the cartridge holder will not be described herein.

In the illustrated embodiment, the cartridge 7 is of the dual-chamber type, including a cylindrical barrel 62 and at the distal end thereof, a neck portion 63. The cartridge 7 further includes two pistons 65, 66 slidably arranged within the cylindrical barrel 62. In the initial state of the cartridge 7, as shown on Figure 3, the first piston 65 is provided at the proximal end of the barrel 62 so as to sealingly close the proximal end of the cartridge 7 and the second piston 66 is arranged distally and axially spaced with respect to the first piston. Both pistons 65, 66 thus define two compartments or chambers 67, 68, typically containing products to be mixed before the injection. In particular, the first chamber 67 typically contains a liquid formulation (or solvent) and the second chamber 68, distally located with respect to the first chamber, contains a lyophilized medicament. The barrel 62 is formed with a by-pass section 69 to allow for the liquid formulation to flow within the second chamber 68 and mix with the lyophilized medicament. The neck portion 63 is formed with a distal opening and provided with a septum 71, that closes the distal opening and can be penetrated by a hollow needle. When the cartridge 7 is received in the cartridge holder 5, the neck portion 63 is engaged within the distally projecting cylindrical sleeve of the cartridge holder that is formed with the needle connection 55.

As can be seen on Figure 10A, the needle assembly 60 includes the needle 61 and a needle hub 73 supporting the needle and provided with an internal thread fitting with the needle connection 55 of the cartridge holder 5 for attachment of the needle 61 to the cartridge, in fluid communication with the chamber 68. The needle assembly 60 further includes a detachable needle cap 75 for protecting the needle 61 while the needle is being attached to the cartridge 7. The needle cap 75 can be removed one the needle 61 is attached to the cartridge 7.

While the illustrated embodiment comprises a dual chamber cartridge, it will be appreciated that the invention is applicable to any type of cartridges, in particular to cartridges having a single compartment and containing liquid product.

Still referring to Figures 1 and 3, the injection drive mechanism 9 comprises an axial and axially displaceable piston rod 81 for driving the pistons 65, 66 in the cartridge 7 and an electrical motor 83, provided with an output shaft 84 and controlled by the electronic control system, for displacing the piston rod 81. The device 1 comprises a battery (not shown) for supplying the motor 83 with electric power. The injection drive mechanism 9 further comprises a gearbox 85 transmitting and converting motion of the output shaft 84 so as to cause linear displacement of the piston rod 81.

The injection drive mechanism 9 is attached to the chassis 3, with the stationary parts of the injection drive mechanism 9, including the motor 83 and the gearbox 85, being rigidly attached to the chassis and the piston rod 81 being axially displaceable with respect to those stationary parts and the chassis 3.

When a needle is attached to the cartridge 7 and when the device is actuated, in an injection mode, the injection drive mechanism 9 causes the piston rod 81 to drive the pistons 65, 66 within the cartridge. With a dual chamber configuration such as illustrated with the presented embodiment, this may cause the liquid formulation to mix with the lyophilized medicament and/or expel medicament from the cartridge through the needle 61. Alternatively, with a single chamber configuration, driving the piston within the cartridge in the proximal direction results in liquid medicament to be expelled from the cartridge through the needle.

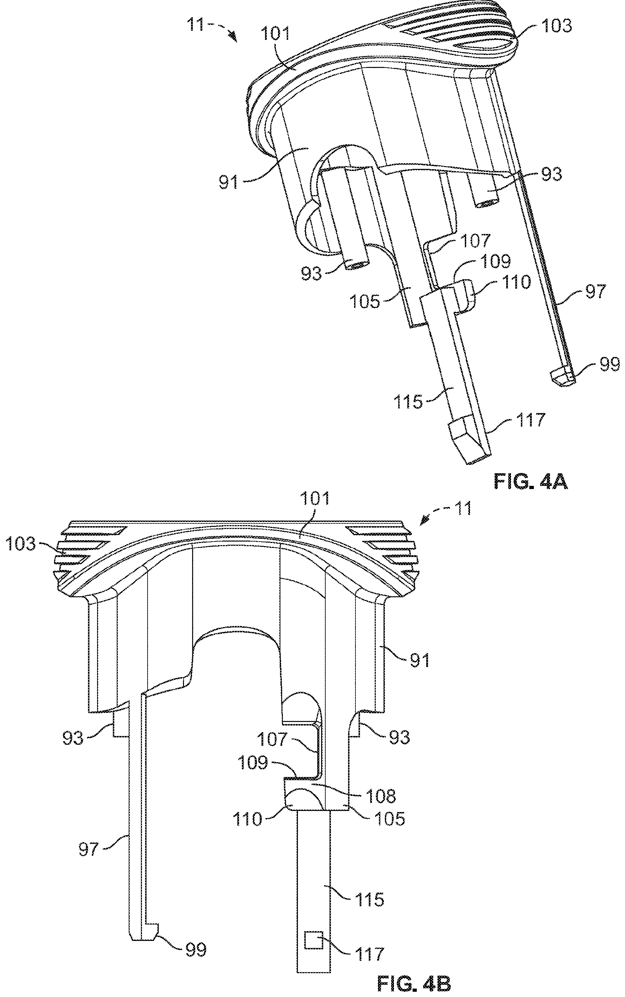

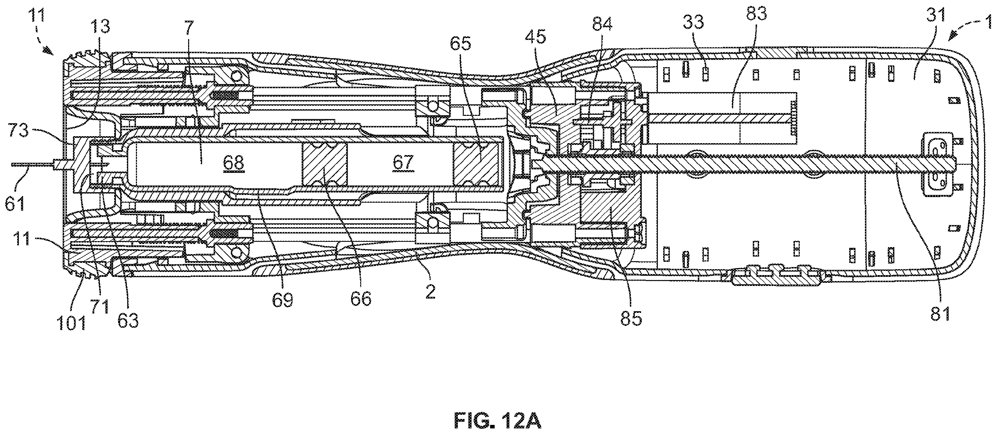

With particular reference to Figures 1, 3, 4A and 4B, the needle hiding element 11 is telescopically mounted in the housing 2 and can be axially displaced between a fully extended position illustrated on Figure 3 and a fully retracted position (Figure 12A). In the fully extended position, the needle hiding element 11 distally protrudes from the housing 2 and is suitable for hiding a needle 61 attached to the cartridge 7. The fully retracted position of the needle hiding element 11 is proximally spaced from the extended position and corresponds to the condition of pressing the device 1 against an injection site for injecting the medicament.

The needle hiding element 11 comprises an essentially cylindrical wall 91 suitable, in the fully extended position, for hiding the needle 61 attached to the medicament cartridge 7.

The needle hiding element 11 further comprises a pair of guiding elements 93 formed as hollow cylinders proximally projecting from the cylindrical wall 91. The pair of hollow cylinders 93 slidably engage with respective guiding studs 41 of a corresponding pair provided on the chassis 3 and defining complementary guiding elements for axially guiding the needle hiding element 11 as it moves between the fully extended position and the fully retracted position. The needle hiding element 11 further comprises biasing means in the form of a pair of compression springs 95 each arranged in a respective hollow cylinder 93 for biasing the needle hiding element 11 into its fully extended position.

The needle hiding element 11 further comprises an axially extending retention arm 97 proximally projecting from the cylindrical wall 91 and provided with a stop member 99 suitable for engaging with a complementary stop member (not shown) of the chassis 3 in the fully extended position to limit the displacement of the needle hiding element 11 in the distal direction. The complementary stop member may be otherwise secured to the housing 2 or alternatively be integral with the housing 2.

The needle hiding element 11 further comprises at the distal end of the cylindrical wall 91 an enlarged gripping portion 101 that radially extends beyond the cylindrical wall. The enlarged gripping portion 101 may be formed with a relatively soft material relatively to the material of the cylindrical wall 91 and the rest of the needle hiding element. It may be either overmolded or attached to the cylindrical wall 91. The gripping portion 101 has a ridged surface 103 for enhancing prehension by a user. The enlarged gripping portion is designed to engage the distal edge 17 of the housing 2 in the fully retracted position of the needle hiding element 11 and thus prevent further retraction. In that fully retracted position, the gripping portion 101 engages, in the sense that it presses against, the distal edge 17 of the housing 2, but does not directly contacts the distal edge 17 as the annular seal 15 is interposed between the distal edge 17 and the gripping portion 101.

The needle hiding element 11 further comprises, proximally projecting from the cylindrical wall 91, an axially extending latching arm 105, provided with a notch 107 open in the transverse direction, a rigid stop member 108 formed with a transversal abutment surface 109 and an inclined ramp 110. The needle hiding element 11 also has, formed as an axial extension of the latching arm 105, a detection arm 115 with a magnetic part, such as a magnet 117, attached thereto.

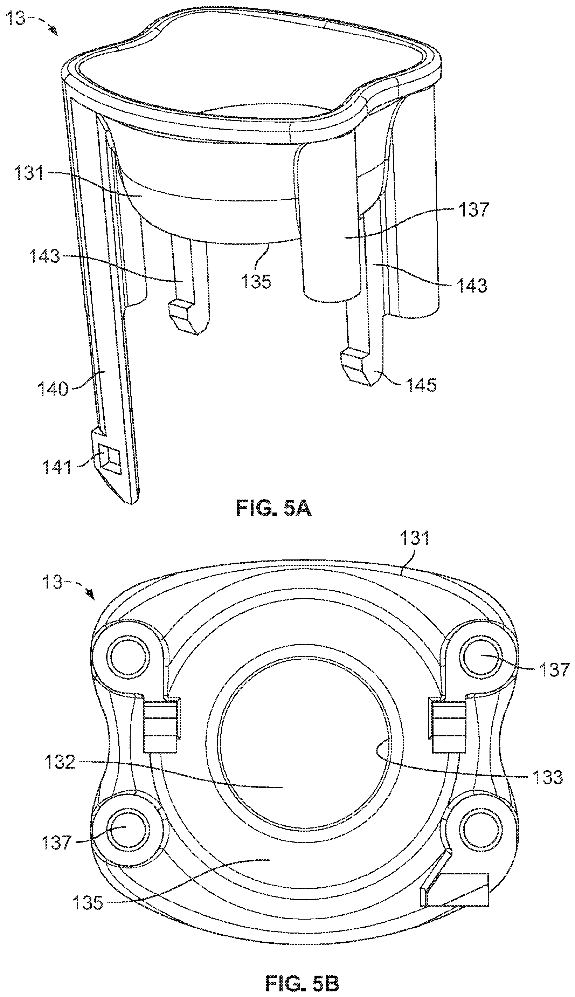

With particular reference to Figures 1, 3 and 5, the slider 13 is telescopically mounted in the housing 2 and is axially movable between an extended position illustrated on Figure 3 and a retracted position (Figure 10A).

The slider 13 comprises a base 131 for engagement with the needle assembly 60 (Figure 10A). The base has a central bore 132 designed such that the needle 61 can pass through the bore while the needle hub 73 engages the base at a peripheral rim 133 of the bore, when a needle assembly 60 is being connected to the needle connection 55. The base 131 of the slider 13 has a cup shape with a bottom wall 135 provided with the bore 132 and proximally oriented. The bottom wall 135 is substantially flat whereby, when the needle hub 73 is threadedly connected to the needle connection 55 i.e. to the threaded section of the cartridge holder 5, with the needle 61 thus attached to the cartridge 7, the bottom wall 135 is tightly maintained between the needle hub 73 and the cartridge holder 5. In this configuration, the slider 13 is maintained in the retracted position and a partial sealing preventing ingress of any expelled medicament from the cartridge 7 into the housing 2 is provided.

Similarly to the needle hiding element 11, the slider 13 comprises a pair of guiding elements formed as hollow cylinders 137 proximally projecting from the base 131. The pair of hollow cylinders 137 slidably engage with respective guiding studs 41 of a corresponding pair provided on the chassis 3 and defining complementary guiding elements for axially guiding the slider 13 as it moves between the extended position and the retracted position. The slider 13 further comprises biasing means in the form of a pair of compression springs 139 each arranged in a respective hollow cylinder 137 and operatively associated therewith for biasing the slider 13 into its extended position.

The slider comprises an axial arm 140 proximally projecting from the base 131 and provided with a magnetic part, such as a slider magnet (not shown) inserted and secured in a cavity 141 located at a free end of the axial arm 140. The slider 13 also comprises a pair of axially extending retention arm 143 proximally projecting from the base 131 and provided with a stop member 145 suitable for engaging with a complementary stop member (not shown) of the chassis 3 in the extended position to limit the displacement of the slider 13 in the distal direction.

It will be noted that the needle hiding element 11 and the slider 13 can slide independently, relatively to the housing 2, between their respective extended and retracted positions.

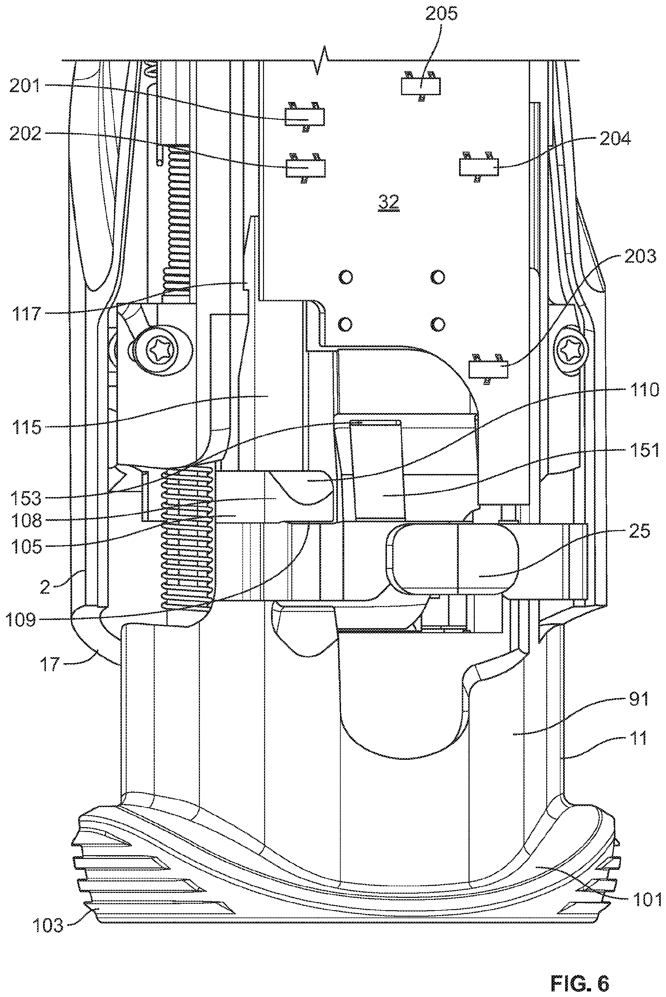

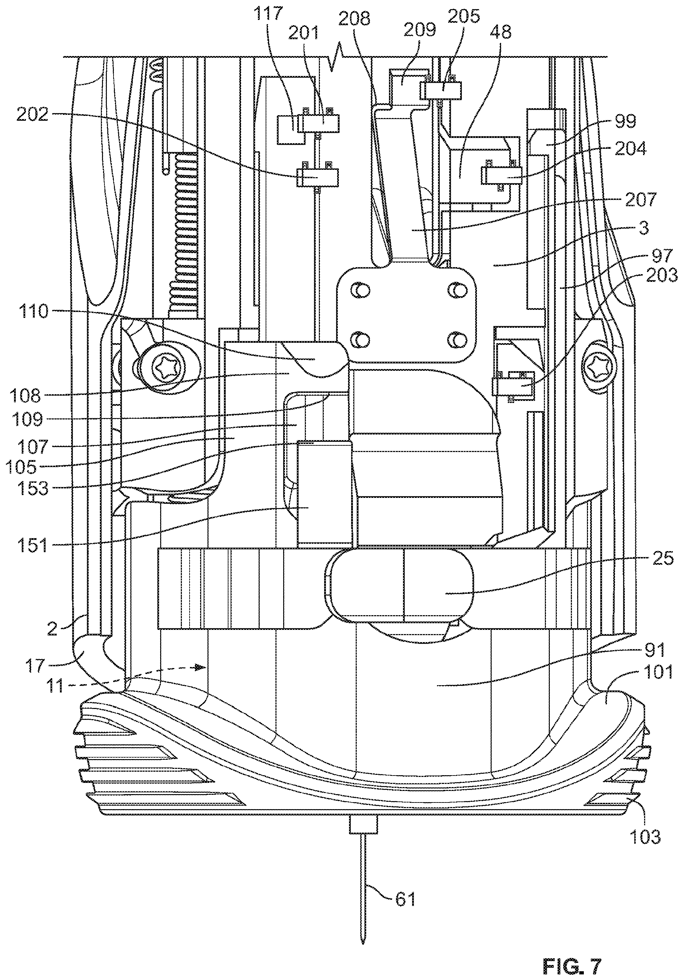

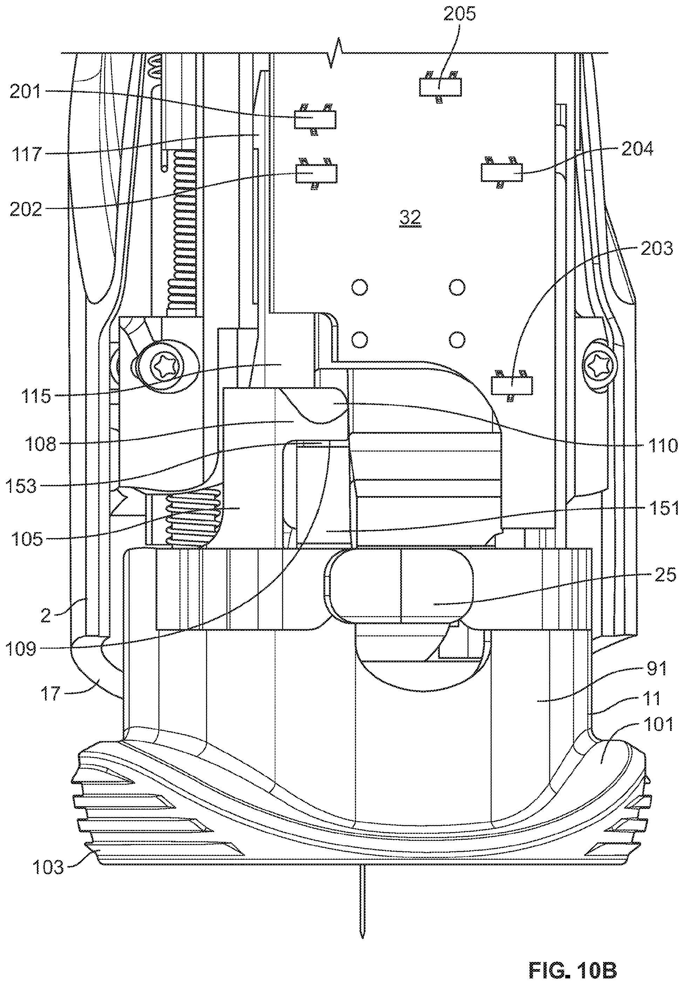

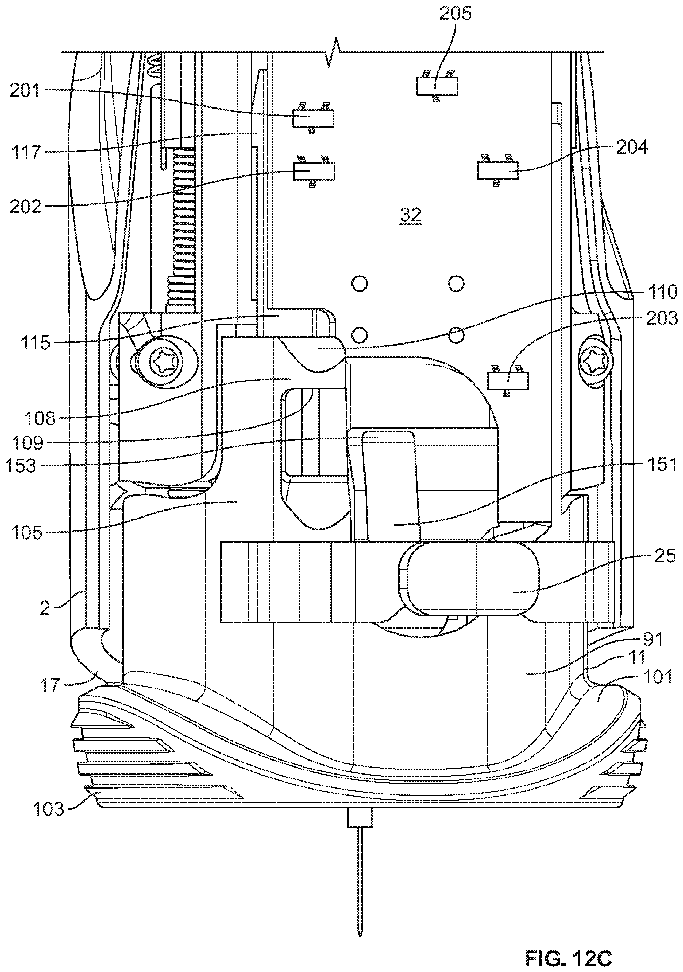

With particular reference to Figures 6, 7 and 10B, it will be seen that the injection device 1 includes a latch mechanism that can be actuated by a user for releasably locking the needle hiding element 11 in one partially retracted position (Figures 10A, 10B), proximally spaced from the fully extended position (Figures 3, 6) and distally spaced from the fully retracted position (Figures 7, 12C). The partially retracted position is suitable for facilitating the attachment of a needle 61 to the cartridge 7.

In the configurations of Figures 6, 7 and 10B, the needle hiding element 11 is respectively in its fully extended position, its fully retracted position and the partially retracted, locked, position.

The latch mechanism includes the latch button 25, that is actuatable by the user to be transversally displaced between a release position and a locking position, a first latch member attached to the latch button 25 and a corresponding second latch member provided on the needle hiding element 11.

In the illustrated embodiment, the first latch member is a flexible arm in the form of a metallic blade 151 extending in an axial direction and attached at one end to the latch button 25. The other end of the blade is a free end having an edge 153 for engagement with the second latch member.

The second latch member is defined by the latching arm 105 and includes the rigid stop member 108 that is formed with the transversal abutment surface 109 and the inclined ramp 110. In the locking position of the latch members, the edge 153 of the blade 151 engages with the transversal abutment surface 109.

It will be understood that the displacement of the needle hiding element 11 defines a sliding path for the latching arm 105, and that the latch mechanism is designed such that the blade 151 is out of the sliding path of the latching arm 105 when the latch button 25 is in the release position, as shown on Figure 6 (also illustrated on Figure 12C). When the latch button 25 is in the locking position, the blade 151 is within the sliding path of the latching arm 105.

The blade 151 is suitable, in the locking position of the latch button 25 and when the needle hiding element 11 is moved proximally from its fully extended position, to engage with and slide over the ramp 110 and flex out to allow for such proximal displacement of the needle hiding element. When reaching the notch 107, the blade 151 flexes back out to fall in the notch 107. If the needle hiding element 11, from that axial position, is moved back distally toward its extended position, the edge 153 engages with the abutment surface 109 thus preventing distal displacement from the partially retracted position of the needle hiding element 11. However, from that axial position, the latch mechanism allows for further proximal displacement of the needle hiding element 11 until the fully retracted position (Figure 7).

In an alternative embodiment (not represented), the flexible arm or blade 151 may be provided on the needle hiding element 11 and the rigid stop member 108 on the latch button 25.

As illustrated by Figures 6, 7, 10B, 12C, the injection device 1 comprises a position sensing feature for detecting the position of the needle hiding element 11 and providing a corresponding information to the electronic control system.

The position sensing feature includes the magnet 117 provided on the detection arm 115 of the needle hiding element 11. It further includes a first 201 and a second 202 electromagnetic sensors supported by the PCB 32 and thus fixedly arranged within the housing 2. The second electromagnetic sensor 202 is axially spaced from the first electromagnetic sensor 201 in the distal direction by a distance corresponding to the travel of the needle hiding element 11 between the fully retracted position (Figure 7) and the partially retracted position (Figure 10B). The electromagnetic sensors 201, 202 are connected to the processor so as to provide to the processor a signal indicative of the position of the needle hiding element 11.

With the needle hiding element 11, the magnet 117 is axially movable between positions remote from the first 201 and second 202 sensors (Figure 6), a position remote from the first sensor 201 and close to the second sensor 202 (Figure 10B), and a position close to the first electromagnetic sensor 201 and remote from the second sensor 202.

When in a position remote from one of the electromagnetic sensors 201, 202, the magnetic field of the magnet 117 is not able to modify the state of that sensor 210, 202. When in a position close to (or in the vicinity of) one of the electromagnetic sensors 201, 202, the magnetic field of the magnet 117 is able to modify the state of that sensor 201, 202.

The position of the magnet 117 close to the first sensor 201 corresponds to a configuration where the needle hiding element 11 is in its fully retracted position. In this position, the first sensor 201 is caused to generate, and provide to the electronic control system, a signal indicative of the injection device 1 being pressed against an injection surface and ready for injection.

The position of the magnet 117 close to the second sensor 202 corresponds to a configuration where the needle hiding element 11 is in its partially retracted position. In this position (if maintained by the latch mechanism), the second sensor 202 is caused to generate, and provide to the electronic control system, a signal indicative of the needle hiding element 11 being in a suitable position for the user to attach a needle to the cartridge.

The electronic control system is adapted to prevent actuation of the injection drive mechanism 9 for expelling medicament from the cartridge, unless it is provided by the first electromagnetic sensor 201 with a signal indicative of the injection device 1 being pressed against an injection surface and ready for injection.

As illustrated by Figures 6, 9C, 10C, the injection device further includes a needle sensing feature for detecting the attachment of a needle 61 to the cartridge 7 and providing a corresponding information to the electronic control system.

The needle sensing feature includes the slider magnet provided on the axial arm 140 of the slider 13 and a third electromagnetic sensor 203 supported by the PCB 32 and thus fixedly arranged within the housing 2. The electromagnetic sensor 203 is connected to the processor so as to provide to the processor a signal indicative of a needle being attached to the cartridge.

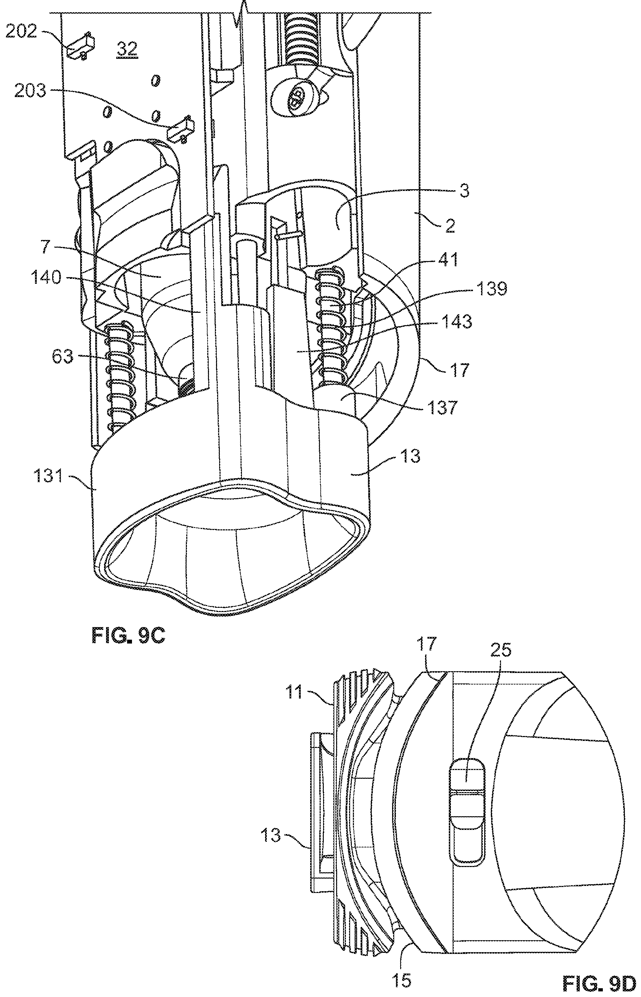

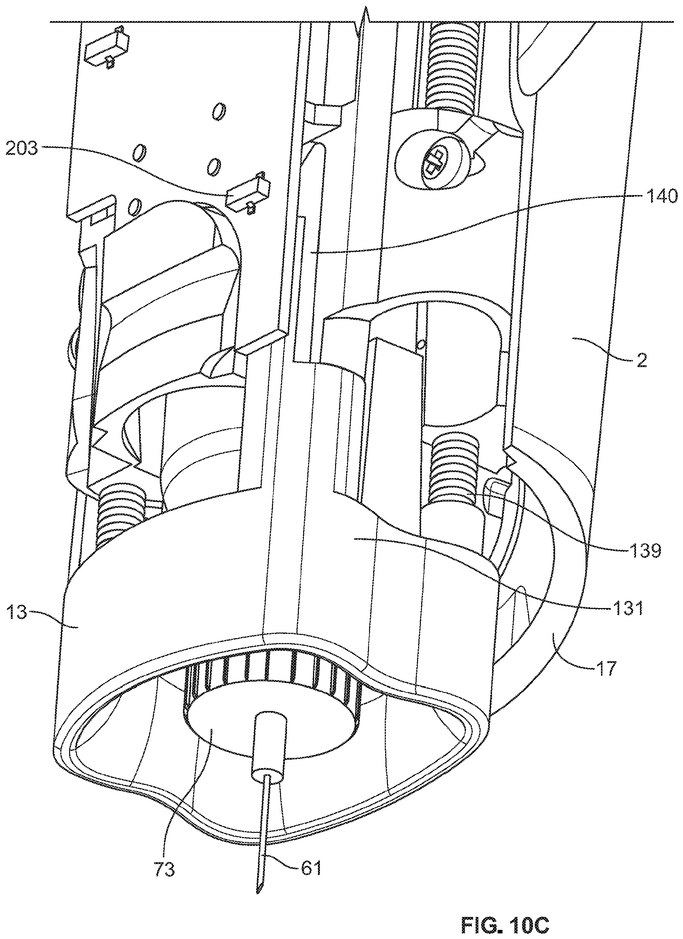

With the slider 13, the slider magnet provided in the cavity 141 is axially movable between positions remote from the sensor 203 (Figure 9C) and a position close to the sensor 203 (Figure 10C). When in a position remote from the electromagnetic sensor 203, the magnetic field of the slider magnet is not able to modify the state of that sensor 203. When in a position close to (or in the vicinity of) the electromagnetic sensors 203, the magnetic field of the slider magnet is able to modify the state of that sensor 203.

The position of the slider magnet remote from the sensor 203 as illustrated on Figure 9C corresponds to the configuration where the slider 13 is in its extended position. The position of the slider magnet close to the sensor 203 as illustrated on Figure 10C corresponds to a configuration where the slider 13 is in its retracted position. The slider 13 being maintained in that retracted position requires that a needle 61 be attached on the cartridge 7. In this position, the sensor 203 is caused to generate, and provide to the electronic control system, a signal indicative of a needle being attached to the cartridge.

The electronic control system is adapted to prevent actuation of the injection drive mechanism 9 for expelling medicament from the cartridge, unless it is provided with a signal indicative of a needle being attached to the cartridge by the electromagnetic sensor 203.

Referring now more specifically to Figures 6 and 7, it will be seen that the injection device 1 further includes a fourth 204 and fifth 205 electromagnetic sensors fixedly supported by the PCB 32 and connected to the processor.

Similarly to the electromagnetic sensors 201, 202, 203 described in the foregoing, the sensors 204 and 205 are able to detect the proximity of respective magnets and provide to the processor respective signals.

The fourth sensor (or door sensor) 204 is associated with the door magnet provided on the bottom wall 48 and detects the closed position of the cartridge holder 5. In said closed position, the sensor 204 is caused to generate, and provide to the electronic control system, a signal indicative of the cartridge holder (door) being closed onto the housing 2.

The fifth sensor (or cartridge detection sensor) 205 is associated with a cartridge detection magnet (not represented) that detects the presence of a cartridge 7 in the cartridge holder 5. As already mentioned in the foregoing and visible on Figure 7, the device 1 comprises a flexible detection arm 207 attached to the chassis 3. The detection arm 207 is formed with an elbow 208 and a free end 209 provided with the cartridge detection magnet. The elbow 208 projects though the elongated hole of the bottom wall 48 into the cartridge holder 5, when the cartridge holder 5 is in its closed position. If a cartridge 7 is not present in the cartridge holder 5, the detection arm 207 will remain in its released state with the cartridge detection magnet being spaced from the sensor 205, whereas in case a cartridge is present in the cartridge holder 5, the elbow 208 will be pushed out and cause the detection arm 207 to flex and the free end 209 to be moved closed to the sensor 205.

In the latter configuration, wherein a cartridge 7 is present in the cartridge holder 5 and the cartridge holder 5 closed onto the housing 2, the sensor 205 is caused to generate, and provide to the electronic control system, a signal indicative of a cartridge being in place in the housing 2 with the door closed.

Starting from an initial configuration of the injection device as shown on Figures 2A and 2B, the main steps for operating the device will now be described with reference to Figures 8 to 12.

A user willing to proceed with an injection needs to ensure that a medicament cartridge 7 is present in the cartridge older 5. This information can be automatically acquired by the device and displayed on the screen 19. If necessary to load a fresh cartridge into the device, the user needs to open the cartridge holder 5 and axially insert the cartridge into the cartridge holder 5 as illustrated on Figure 8. The reverse operation consisting in removing an empty cartridge from the device, in particular for replacing it, can also be illustrated by the same Figure 8.

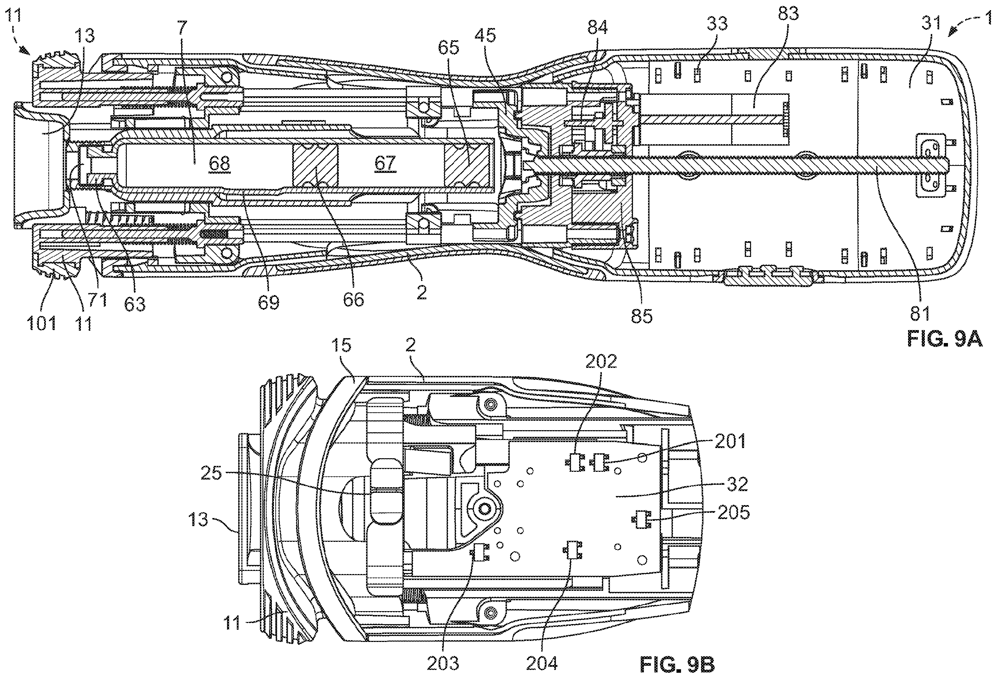

The sequence of operations for locking the needle hiding feature 11 and attaching a needle to the cartridge will now be described with reference to Figures 9A, 9B, 9C, 9D, 10A and 10B.

The user needs to manually retract the needle hiding element 11 from the extended position and lock it in the partially retracted position (Figure 9A, 9B, 9D) suitable for placing the needle assembly. To that end, the user needs to transversally move the latch button 25 into the position shown on Figures 9B, 9D. As explained in the foregoing, the user may either put the button 25 in the locking position before retracting the needle hiding element 11 or after. If the button 25 is in the locked position before retraction, the needle hiding element 11 can be retracted as the flexible blade can slide on the ramp 110 during retraction and the latch members can reach their engagement position. Since the needle hiding element 11 and the slider can move independently, the slider 13 remains in its extended position.

In particular, as shown on Figure 9C, the slider magnet remains axially spaced from the sensor 203.

Figure 10A illustrates the step of attaching a needle 61 to the cartridge 7. For doing so, the user needs to insert the needle assembly 60 into the distal end of the device, thereby pushing back the slider 13 in the proximal direction. The attachment is achieved by threadedly engaging the needle hub 73 onto the needle connection 55 of the cartridge holder 5. In doing so, the needle 61 penetrates the septum 71. The user may then remove the needle cap 75, thereby exposing the needle.

In this position, as shown on Figure 10B, the magnet 117 of the needle hiding element 11 is closed to the sensor 202, thus indicating to the electronic control system that the needle hiding element 11 is in suitable locked position for the needle to be attached.

With the slider 13 maintained in the retracted position by the needle hub 73, the slider magnet remains in a position close to the sensor 203 indicative of the presence of a needle 61 attached to the cartridge, as illustrated on Figure 10C.

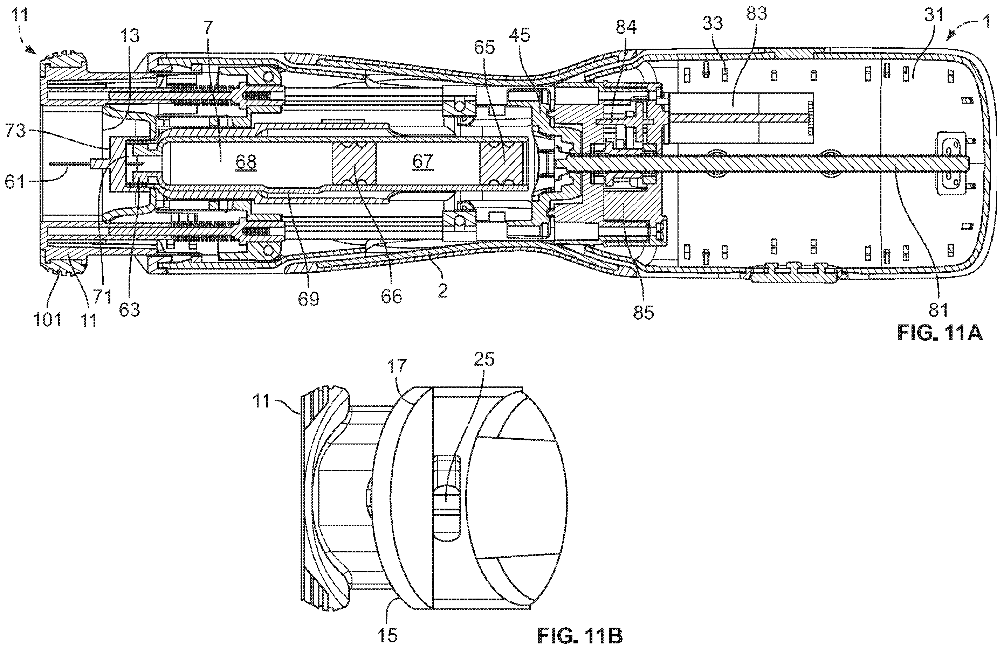

Once the needle 61 attached to the cartridge, the user may move the latch button 25 back to the release position (Figure 11B) so as to release the latch mechanism and allow the needle hiding element 11 to return to its initial extended position (Figures 11A and 11B) under the biasing effect of the springs 95. In that position, the cylindrical wall 91 of the needle hiding element surrounds the needle 61, thereby hiding the needle and helping reducing the anxiety associated with the injection. The device is ready for an injection.

It will be appreciated that, in that position, the magnet 117 of the needle hiding element 11 is remote from the sensors 201, 202, as shown on Figure 6.

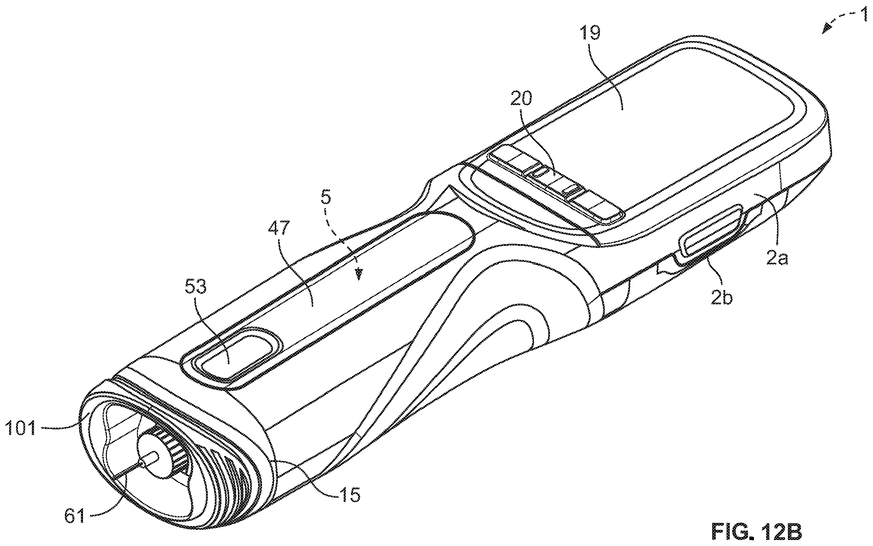

Figures 12A, 12B and 12C illustrate the injection device 1 in the injection configuration, wherein the user presses the device against the injection site in view of triggering the injection of medicament from the cartridge by pressing the injection button 22.

As visible on those Figures, the needle hiding element 11 is moved into its fully retracted position against the biasing force of the springs 95 and the gripping portion 101, bearing against the annular seal 15 (not shown on Figure 12C), is stopped by the distal edge 17 of the housing. The slider 13 is still maintained in the retracted position.

In this position, as shown on Figure 12C, the magnet 117 of the needle hiding element 11 is closed to the first sensor 201, thus indicating to the electronic control system that the needle hiding element 11 is in the fully retracted position and in a suitable position for the injection to be performed.

It will be appreciated that the electronic control system is set to control the injection drive mechanism for expelling medicament from the cartridge subject to cumulative multiple conditions, that may include one or more of the following:
- cartridge holder closed;
- cartridge present in the cartridge holder;
- needle properly attached to the cartridge; and
- needle hiding element fully retracted.

## Claims

1. Injection device comprising
- an elongated housing (2) extending along a longitudinal axis (X) and configured to receive a medicament cartridge (7);
- a needle connection (55) for connection to a needle assembly (60), including a needle (61) and a needle hub (73), and thus attaching the needle to the cartridge;
- an injection drive mechanism (9) for driving a piston (65, 66) within the cartridge and thus expel medicament from the cartridge (7);
- an electronic control system for controlling the injection drive mechanism (9) based on user input and information indicative of the status of the device (1); and
- a needle sensing feature for detecting the attachment of a needle (61) to the cartridge (7) and providing a corresponding information to the electronic control system,
**characterised in that** the needle sensing feature includes
- a slider (13) which is axially movable relative to the housing between an extended position and a retracted position, the slider (13) comprising a magnetic part and being biased into its extended position and maintained in its retracted position when a needle (61) is attached to the cartridge (7);
- an electromagnetic sensor (203) fixedly arranged within the housing (2), such that the magnetic part can move with the slider (13) between positions remote from the electromagnetic sensor (203) and a position in the vicinity of the electromagnetic sensor when the slider (13) is in its retracted position, wherein the electromagnetic sensor (203) is caused to generate, and provide to the electronic control system, a signal indicative of a needle (61) being attached to the cartridge (7).

2. Injection device according to claim 1, wherein the electronic control system is adapted to prevent actuation of the injection drive mechanism (9) for expelling medicament from the cartridge (7), unless it is provided with a signal indicative of a needle (61) being attached to the cartridge (7) by the electromagnetic sensor (203).

3. Injection device according to claim 1 or 2, wherein the injection drive mechanism (9) comprises an axially displaceable piston rod (81) for driving a piston (65, 66) in the cartridge (7) and an electrical motor (83) for displacing the piston rod (81), the electrical motor (83) being controlled by the electronic control system.

4. Injection device according to any one of claims 1 to 3, further comprising a chassis (3) arranged within the housing (2) and fixedly attached thereto, and wherein the injection drive mechanism (9) is attached to the chassis (3).

5. Injection device according to claim 4, wherein the electronic control system includes a processor and a printed circuit board (32) fixedly arranged within the housing (2), wherein the electromagnetic sensor (203) is supported by the printed circuit board (32) and connected to the processor so as to provide to the processor a signal indicative of a needle (61) being attached to the cartridge (7).

6. Injection device according to any one of claims 1 to 5, further comprising a cartridge holder (5) for receiving and holding the cartridge (7), the cartridge holder (5) being movable relative to the housing (2) between an open position, wherein a cartridge (7) can be placed within or removed from the cartridge holder (5), and a closed position, wherein the cartridge (7) is in a position suitable for injection.

7. Injection device according to claim 6, wherein the cartridge holder (5) is pivotable between the open position and the closed position.

8. Injection device according to claims 4 and 7, wherein the chassis (3) and the cartridge holder (5) comprise fitting pivot pins (51) and pivot bearings (39) forming a hinge connection, whereby the cartridge holder (5) is pivotable with respect to the housing (2) between the open and the closed position.

9. Injection device according to any one of claims 6 to 8, wherein the needle connection (55) comprises a threaded tubular section of the cartridge holder (5) provided at a distal end thereof for threaded connection to the needle hub (73).

10. Injection device according to any one of claims 1 to 9, wherein the slider (13) comprises a base (131) for engagement with the needle assembly (60), the base (131) having a central bore (132) designed such that the needle (61) can pass through the bore (132) while the needle hub (73) engages the base (131) at a peripheral rim (133) of the bore (132) when a needle assembly (60) is being connected to the needle connection (55).

11. Injection device according to claim 10, wherein the base (131) of the slider (13) has a cup shape with a bottom wall provided with the bore (132) and proximally oriented, the bottom wall being substantially flat whereby, when the needle hub (73) is threadedly connected to the threaded section of the cartridge holder (5) and the needle (61) thus attached to the cartridge (7), the bottom wall is tightly maintained in the retracted position of the slider (13) between the needle hub (73) and the cartridge holder (5), thereby providing partial sealing preventing ingress of any expelled medicament from the cartridge (7) into the housing (2).

12. Injection device according to claim 10 or 11, wherein the slider (13) comprises an axial arm (140) proximally projecting from the base (131) and provided with the magnetic part.

13. Injection device according to any one of claims 10 to 12, wherein the slider (13) comprises guiding elements (137) proximally projecting from the base (131) for axially guiding the slider (13) as it moves between the extended position and the retracted position.

14. Injection device according to claims 4 and 13, wherein the chassis (3) comprises complementary guiding elements (41) engaging with respective guiding elements (137) of the slider (13) for axially guiding the slider (13) as it moves between the extended position and the retracted position.

15. Injection device according to claim 13 or 14, wherein the slider (13) comprises biasing springs operatively associated with the respective guiding elements (137) for biasing the slider (13) into its extended position.

16. The injection device according to claim 1, wherein, in the retracted position, the slider is configured to be maintained between a threaded connection formed between the needle hub and the needle connection.

## Patentansprüche

1. Injektionsvorrichtung, umfassend
• ein längliches Gehäuse (2), das sich entlang einer Längsachse (*X*) erstreckt und zur Aufnahme einer Medikamentenkartusche (7) konfiguriert ist;
• eine Nadelverbindung (55) zur Verbindung mit einer Nadelanordnung (60), die eine Nadel (61) und einen Nadelansatz (73) aufweist, und somit zum Befestigen der Nadel an der Kartusche;
• einen Injektionsantriebsmechanismus (9) zum Antreiben eines Kolbens (65, 66) innerhalb der Kartusche und somit zum Ausstoßen von Medikament aus der Kartusche (7);
• ein elektronisches Steuersystem zum Steuern des Injektionsantriebsmechanismus (9) basierend auf einer Benutzereingabe und Informationen, die den Zustand der Vorrichtung (1) anzeigen; und
• eine Nadelerfassungseinrichtung zum Erfassen der Befestigung einer Nadel (61) an der Kartusche (7) und zum Bereitstellen einer entsprechenden Information an das elektronische Steuersystem,
**dadurch gekennzeichnet, dass** die Nadelerfassungseinrichtung Folgendes aufweist:
• einen Schieber (13), der relativ zum Gehäuse zwischen einer ausgefahrenen Position und einer zurückgezogenen Position axial bewegbar ist, wobei der Schieber (13) ein magnetisches Teil aufweist und in seine ausgefahrene Position vorgespannt ist und in seiner zurückgezogenen Position gehalten wird, wenn eine Nadel (61) an der Kartusche (7) befestigt ist;
• einen elektromagnetischen Sensor (203), der fest innerhalb des Gehäuses (2) angeordnet ist, so dass sich das magnetische Teil mit dem Schieber (13) zwischen Positionen fern von dem elektromagnetischen Sensor (203) und einer Position in der Nähe des elektromagnetischen Sensors bewegen kann, wenn sich der Schieber (13) in seiner zurückgezogenen Position befindet, wobei der elektromagnetische Sensor (203) veranlasst wird, ein Signal zu erzeugen und an das elektronische Steuersystem bereitzustellen, das anzeigt, dass eine Nadel (61) an der Kartusche (7) befestigt ist.

2. Injektionsvorrichtung nach Anspruch 1, wobei das elektronische Steuersystem so angepasst ist, dass es eine Betätigung des Injektionsantriebsmechanismus (9) zum Ausstoßen von Medikament aus der Kartusche (7) verhindert, es sei denn, es wird ihm durch den elektromagnetischen Sensor (203) ein Signal bereitgestellt, das anzeigt, dass eine Nadel (61) an der Kartusche (7) befestigt ist.

3. Injektionsvorrichtung nach Anspruch 1 oder 2, wobei der Injektionsantriebsmechanismus (9) eine axial verschiebbare Kolbenstange (81) zum Antreiben eines Kolbens (65, 66) in der Kartusche (7) und einen elektrischen Motor (83) zum Verschieben der Kolbenstange (81) aufweist, wobei der elektrische Motor (83) durch das elektronische Steuersystem gesteuert wird.

4. Injektionsvorrichtung nach einem der Ansprüche 1 bis 3, ferner umfassend ein Chassis (3), das innerhalb des Gehäuses (2) angeordnet und fest daran befestigt ist, und wobei der Injektionsantriebsmechanismus (9) an dem Chassis (3) befestigt ist.

5. Injektionsvorrichtung nach Anspruch 4, wobei das elektronische Steuersystem einen Prozessor und eine Leiterplatte (32) aufweist, die fest innerhalb des Gehäuses (2) angeordnet ist, wobei der elektromagnetische Sensor (203) von der Leiterplatte (32) getragen wird und mit dem Prozessor verbunden ist, um dem Prozessor ein Signal bereitzustellen, das anzeigt, dass eine Nadel (61) an der Kartusche (7) befestigt ist.

6. Injektionsvorrichtung nach einem der Ansprüche 1 bis 5, ferner umfassend einen Kartuschenhalter (5) zur Aufnahme und zum Halten der Kartusche (7), wobei der Kartuschenhalter (5) relativ zum Gehäuse (2) zwischen einer offenen Position, in der eine Kartusche (7) in den Kartuschenhalter (5) eingesetzt oder daraus entfernt werden kann, und einer geschlossenen Position, in der sich die Kartusche (7) in einer für die Injektion geeigneten Position befindet, bewegbar ist.

7. Injektionsvorrichtung nach Anspruch 6, wobei der Kartuschenhalter (5) zwischen der offenen Position und einer geschlossenen Position verschwenkbar ist.

8. Injektionsvorrichtung nach den Ansprüchen 4 und 7, wobei das Chassis (3) und der Kartuschenhalter (5) passende Drehbolzen (51) und Schwenklager (39) aufweisen, die eine Scharnierverbindung bilden, wodurch der Kartuschenhalter (5) in Bezug auf das Gehäuse (2) zwischen der offenen und der geschlossenen Position verschwenkbar ist.

9. Injektionsvorrichtung nach einem der Ansprüche 6 bis 8, wobei die Nadelverbindung (55) einen rohrförmigen Gewindeabschnitt des Kartuschenhalters (5) aufweist, der an einem distalen Ende davon zur Gewindeverbindung mit dem Nadelansatz (73) vorgesehen ist.

10. Injektionsvorrichtung nach einem der Ansprüche 1 bis 9, wobei der Schieber (13) eine Basis (131) zum Eingriff mit der Nadelanordnung (60) aufweist, wobei die Basis (131) eine zentrale Bohrung (132) aufweist, die so ausgelegt ist, dass die Nadel (61) durch die Bohrung (132) hindurchgehen kann, während der Nadelansatz (73) an einem peripheren Rand (133) der Bohrung (132) an der Basis (131) angreift, wenn eine Nadelanordnung (60) mit der Nadelverbindung (55) verbunden wird.

11. Injektionsvorrichtung nach Anspruch 10, wobei die Basis (131) des Schiebers (13) eine Becherform mit einer Bodenwand aufweist, die mit der Bohrung (132) versehen und proximal ausgerichtet ist, wobei die Bodenwand im Wesentlichen flach ist, wodurch, wenn der Nadelansatz (73) mit dem Gewindeabschnitt des Kartuschenhalters (5) verschraubt und die Nadel (61) somit an der Kartusche (7) befestigt ist, die Bodenwand in der zurückgezogenen Position des Schiebers (13) fest zwischen dem Nadelansatz (73) und dem Kartuschenhalter (5) gehalten wird, wodurch eine teilweise Abdichtung bereitgestellt wird, die ein Eindringen von ausgestoßenem Medikament aus der Kartusche (7) in das Gehäuse (2) verhindert.

12. Injektionsvorrichtung nach Anspruch 10 oder 11, wobei der Schieber (13) einen axialen Arm (140) aufweist, der proximal von der Basis (131) vorsteht und mit dem magnetischen Teil versehen ist.

13. Injektionsvorrichtung nach einem der Ansprüche 10 bis 12, wobei der Schieber (13) Führungselemente (137) aufweist, die proximal von der Basis (131) vorstehen, um den Schieber (13) axial zu führen, wenn er sich zwischen einer ausgefahrenen Position und einer zurückgezogenen Position bewegt.

14. Injektionsvorrichtung nach den Ansprüchen 4 und 13, wobei das Chassis (3) komplementäre Führungselemente (41) aufweist, die mit jeweiligen Führungselementen (137) des Schiebers (13) in Eingriff stehen, um den Schieber (13) axial zu führen, wenn er sich zwischen der ausgefahrenen Position und der zurückgezogenen Position bewegt.

15. Injektionsvorrichtung nach Anspruch 13 oder 14, wobei der Schieber (13) Vorspannfedern aufweist, die den jeweiligen Führungselementen (137) betriebsmäßig zugeordnet sind, um den Schieber (13) in seine ausgefahrene Position vorzuspannen.

16. Injektionsvorrichtung nach Anspruch 1, wobei der Schieber in der zurückgezogenen Position so konfiguriert ist, dass er zwischen einer Gewindeverbindung gehalten wird, die zwischen dem Nadelansatz und der Nadelverbindung gebildet ist.

## Revendications

1. Dispositif d'injection comprenant
• un boîtier allongé (2) s'étendant le long d'un axe longitudinal (*X*) et configuré pour recevoir une cartouche de médicament (7) ;
• un raccord d'aiguille (55) pour le raccordement à un ensemble d'aiguille (60), comprenant une aiguille (61) et une embase d'aiguille (73), et fixant ainsi l'aiguille à la cartouche ;
• un mécanisme d'entraînement d'injection (9) pour entraîner un piston (65, 66) à l'intérieur de la cartouche et ainsi expulser le médicament de la cartouche (7) ;
• un système de commande électronique pour commander le mécanisme d'entraînement d'injection (9) sur la base d'une entrée d'utilisateur et d'informations indicatrices de l'état du dispositif (1) ; et
• un élément de détection d'aiguille pour détecter la fixation d'une aiguille (61) à la cartouche (7) et fournir une information correspondante au système de commande électronique,
**caractérisé en ce que** l'élément de détection d'aiguille comprend
• un glisseur (13) qui est mobile axialement par rapport au boîtier entre une position déployée et une position rétractée, le glisseur (13) comprenant une partie magnétique et étant sollicité dans sa position déployée et maintenu dans sa position rétractée lorsqu'une aiguille (61) est fixée à la cartouche (7) ;
• un capteur électromagnétique (203) disposé de manière fixe à l'intérieur du boîtier (2), de sorte que la partie magnétique peut se déplacer avec le glisseur (13) entre des positions éloignées du capteur électromagnétique (203) et une position au voisinage du capteur électromagnétique lorsque le glisseur (13) est dans sa position rétractée, dans lequel le capteur électromagnétique (203) est amené à générer, et à fournir au système de commande électronique, un signal indicateur d'une aiguille (61) fixée à la cartouche (7).

2. Dispositif d'injection selon la revendication 1, dans lequel le système de commande électronique est adapté pour empêcher l'actionnement du mécanisme d'entraînement d'injection (9) pour expulser le médicament de la cartouche (7), à moins qu'un signal indicateur d'une aiguille (61) fixée à la cartouche (7) ne lui soit fourni par le capteur électromagnétique (203).

3. Dispositif d'injection selon la revendication 1 ou 2, dans lequel le mécanisme d'entraînement d'injection (9) comprend une tige de piston (81) déplaçable axialement pour entraîner un piston (65, 66) dans la cartouche (7) et un moteur électrique (83) pour déplacer la tige de piston (81), le moteur électrique (83) étant commandé par le système de commande électronique.

4. Dispositif d'injection selon l'une quelconque des revendications 1 à 3, comprenant en outre un châssis (3) disposé à l'intérieur du boîtier (2) et attaché de manière fixe à celui-ci, et dans lequel le mécanisme d'entraînement d'injection (9) est fixé au châssis (3).

5. Dispositif d'injection selon la revendication 4, dans lequel le système de commande électronique comprend un processeur et une carte de circuit imprimé (32) disposés de manière fixe à l'intérieur du boîtier (2), dans lequel le capteur électromagnétique (203) est supporté par la carte de circuit imprimé (32) et connecté au processeur de manière à fournir au processeur un signal indicateur d'une aiguille (61) fixée à la cartouche (7).

6. Dispositif d'injection selon l'une quelconque des revendications 1 à 5, comprenant en outre un support de cartouche (5) pour recevoir et maintenir la cartouche (7), le support de cartouche (5) étant mobile par rapport au boîtier (2) entre une position ouverte, dans laquelle une cartouche (7) peut être placée à l'intérieur ou retirée du support de cartouche (5), et une position fermée, dans laquelle la cartouche (7) est dans une position appropriée pour l'injection.

7. Dispositif d'injection selon la revendication 6, dans lequel le support de cartouche (5) est pivotant entre la position ouverte et la position fermée.

8. Dispositif d'injection selon les revendications 4 et 7, dans lequel le châssis (3) et le support de cartouche (5) comprennent des axes de pivotement (51) et des paliers de pivotement (39) complémentaires formant une liaison articulée, moyennant quoi le support de cartouche (5) est pivotant par rapport au boîtier (2) entre la position ouverte et la position fermée.

9. Dispositif d'injection selon l'une quelconque des revendications 6 à 8, dans lequel le raccord d'aiguille (55) comprend une section tubulaire filetée du support de cartouche (5) prévue à une extrémité distale de celui-ci pour un raccordement fileté à l'embase d'aiguille (73).

10. Dispositif d'injection selon l'une quelconque des revendications 1 à 9, dans lequel le glisseur (13) comprend une base (131) pour s'engager avec l'ensemble d'aiguille (60), la base (131) ayant un alésage central (132) conçu de telle sorte que l'aiguille (61) peut passer à travers l'alésage (132) tandis que l'embase d'aiguille (73) s'engage avec la base (131) au niveau d'un bord périphérique (133) de l'alésage (132) lorsqu'un ensemble d'aiguille (60) est en cours de raccordement au raccord d'aiguille (55).

11. Dispositif d'injection selon la revendication 10, dans lequel la base (131) du glisseur (13) a une forme de coupelle avec une paroi de fond munie de l'alésage (132) et orientée de manière proximale, la paroi de fond étant sensiblement plane, moyennant quoi, lorsque l'embase d'aiguille (73) est raccordée par filetage à la section filetée du support de cartouche (5) et l'aiguille (61) ainsi fixée à la cartouche (7), la paroi de fond est maintenue fermement dans la position rétractée du glisseur (13) entre l'embase d'aiguille (73) et le support de cartouche (5), assurant ainsi une étanchéité partielle empêchant la pénétration dans le boîtier (2) de tout médicament expulsé de la cartouche (7).

12. Dispositif d'injection selon la revendication 10 ou 11, dans lequel le glisseur (13) comprend un bras axial (140) faisant saillie de manière proximale depuis la base (131) et pourvu de la partie magnétique.

13. Dispositif d'injection selon l'une quelconque des revendications 10 à 12, dans lequel le glisseur (13) comprend des éléments de guidage (137) faisant saillie de manière proximale depuis la base (131) pour guider axialement le glisseur (13) lorsqu'il se déplace entre la position déployée et la position rétractée.

14. Dispositif d'injection selon les revendications 4 et 13, dans lequel le châssis (3) comprend des éléments de guidage complémentaires (41) s'engageant avec des éléments de guidage (137) respectifs du glisseur (13) pour guider axialement le glisseur (13) lorsqu'il se déplace entre la position déployée et la position rétractée.

15. Dispositif d'injection selon la revendication 13 ou 14, dans lequel le glisseur (13) comprend des ressorts de rappel associés de manière fonctionnelle aux éléments de guidage (137) respectifs pour solliciter le glisseur (13) vers sa position déployée.

16. Dispositif d'injection selon la revendication 1, dans lequel, dans la position rétractée, le glisseur est configuré pour être maintenu entre une liaison filetée formée entre l'embase d'aiguille et le raccord d'aiguille.
